# EUROPEAN PATENT APPLICATION

(11) **EP 4 338 665 A1**
(43) Date of publication of application: **20.03.2024**
(21) Application number: 22807024.9
(22) Date of filing: 01.02.2022
(51) Int. Cl.: A61B 5/091

(54) **RESPIRATION MEASUREMENT METHOD, RESPIRATION MEASUREMENT DEVICE, AND RESPIRATION MEASUREMENT SYSTEM**

(30) Priority: 13.05.2021 JP 2021081478
(71) Applicant: Shimadzu Corporation, Nakagyo-ku, Kyoto-shi, Kyoto 604-8511 (JP)
(72) Inventor: IMAI, Shinji, Kyoto-shi, Kyoto 604-8511 (JP); MURATA, Koichi, Kyoto-shi, Kyoto 604-8511 (JP); FURUTA, Masafumi, Kyoto-shi, Kyoto 604-8511 (JP)
(74) Representative: Kilian Kilian & Partner mbB
(86) International application number: PCT/JP2022/003709
(87) International publication number: WO 2022/239317

(57) **Abstract**

A respiratory measurement method includes acquiring respiration signals by a detector (130) attached to a human body, extracting, from the acquired respiration signals, a first signal and a second signal indicating an expiratory phase, and calculating information corresponding to an amount of ventilation by respiration on the basis of the extracted first signal and second signal. The calculating includes performing, for each of the respiration signals, different operation depending upon whether the signal is the first signal or the second signal.

## Description

### TECHNICAL FIELD

The present disclosure relates to a respiratory measurement method, a respiratory measurement device, and a respiratory measurement system.

### BACKGROUND ART

In related art, technique of measuring a respiratory condition has been developed. For example, the following PTL 1 discloses a spirometer capable of directly measuring an amount of ventilation by respiration by breathing into a mouthpiece.

Further, as a method for easily measuring a respiratory condition, for example, the following US Patent No. 7,789,837 (PTL 1) discloses a method for measuring respiratory sound with a microphone.

### CITATION LIST

### PATENT LITERATURE

PTL 1: US Patent No. 7,789,837

### NON PATENT LITERATURE

NPL 1: Fukuda Denshi Co., Ltd., Electronic diagnosis spirometer, SP-370COPD Lung Per/Lung Per plus, https://www.fukuda.co.jp/medical/products/pulmonary/sp _370.html

### SUMMARY OF INVENTION

### TECHNICAL PROBLEM

There are increased needs for grasping information as to how an amount of ventilation changes in an environment where a subject can freely move, such as in daily life, in measurement of a respiratory condition. However, the above-described spirometer performs measurement in a state where the subject puts a mouthpiece in his/her mouth, and thus, it is difficult to measure the amount of ventilation in an environment where the subject can freely move.

While with a method for measuring respiratory sound with a microphone, the number of times of respiration can be grasped by confirming a signal waveform of the measured respiratory sound, information corresponding to an amount of ventilation is not grasped.

An object of the present disclosure, which has been made to solve the problems as described above, is to provide a technique capable of easily grasping information corresponding to an amount of ventilation also in an environment where a subject can freely move.

### SOLUTION TO PROBLEM

A respiratory measurement method according to one aspect of the present disclosure includes acquiring respiration signals by a detector attached to a human body, extracting, from the acquired respiration signals, a first signal and a second signal indicating an expiratory phase, and calculating information corresponding to an amount of ventilation by respiration on the basis of the extracted first signal and second signal. The calculating includes performing, for each of the respiration signals, different operation depending upon whether the signal is the first signal or the second signal.

A respiratory measurement device according to another aspect of the present disclosure includes a detector that acquires respiration signals in a state where the detector is attached to a human body, and a controller that processes the respiration signals. The controller extracts, from the acquired respiration signals by the detector, a first signal indicating an inspiratory phase and a second signal indicating an expiratory phase. The controller calculates information corresponding to an amount of ventilation by respiration on the basis of the extracted first signal and second signal. The controller performs, for each of the respiration signals, different operation depending upon whether the signal is the first signal or the second signal.

A respiratory measurement system according to still another aspect of the present disclosure includes the respiratory measurement device and a display device. The controller outputs the calculated information corresponding to the amount of ventilation. The display device displays the output information corresponding to the amount of ventilation.

### ADVANTAGEOUS EFFECTS OF INVENTION

According to a respiratory measurement method according to the present disclosure, different operation is performed, for each of the respiration signals, depending upon whether the signal is a first signal indicating an inspiratory phase or a second signal indicating an expiratory phase. This makes it possible to easily grasp information corresponding to an amount of ventilation also in an environment where a subject can freely move.

### BRIEF DESCRIPTION OF DRAWINGS

Fig. 1 is a view illustrating an example of an overall configuration of a respiratory measurement system according to a first embodiment.
Fig. 2 is a graph indicating an example of temporal change of respiration signals.
Fig. 3A is a view for explaining integrated values of absolute values of respiration signals and a method for correcting the integrated values.
Fig. 3B is a view for explaining integrated values of absolute values of respiration signals and a method for correcting the integrated values.
Fig. 3C is a view for explaining integrated values of absolute values of respiration signals and a method for correcting the integrated values.
Fig. 4 is a graph indicating an example of temporal change of ventilation amount information before correction.
Fig. 5 is a graph indicating an example of temporal change of ventilation amount information after correction.
Fig. 6 is a flowchart for explaining data processing.
Fig. 7 is a flowchart for explaining ventilation amount information calculation processing.
Fig. 8 is a flowchart for explaining data processing according to a modification.
Fig. 9 is a view illustrating an example of an overall configuration of a respiratory measurement system according to a second embodiment.
Fig. 10 is a view illustrating an example of a measurement device.
Fig. 11 is a view for explaining a state where the measurement device is worn on a human body.
Fig. 12 is a view illustrating a display example of output information.

### DESCRIPTION OF EMBODIMENTS

Embodiments of the present invention will be described in detail below with reference to the drawings. Note that the same reference numerals will be assigned to the same or corresponding portions in the drawings, and redundant description will be omitted.

### [First embodiment]

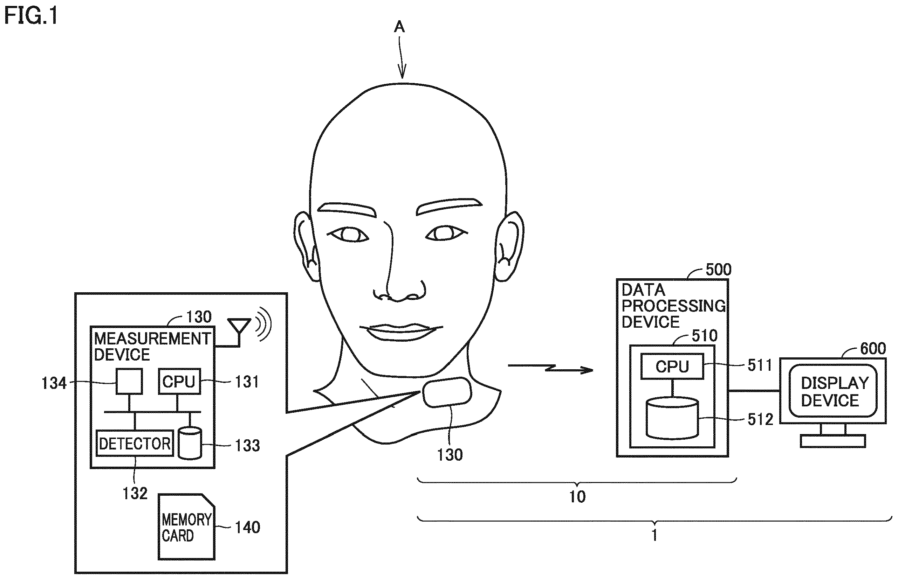

First, a respiratory measurement system 1 according to a first embodiment will be described. Fig. 1 is a view illustrating an example of an overall configuration of respiratory measurement system 1 according to the first embodiment. Respiratory measurement system 1 calculates information corresponding to an amount of ventilation on the basis of the acquired respiration signals and displays the information at a display device 600.

Respiratory measurement system 1 includes a respiratory measurement device 10 and display device 600. Respiratory measurement device 10 includes a data processing device 500 and a measurement device 130. Measurement device 130 includes a detector 132, a central processing unit (CPU) 131, a storage 133, and a transmitter 134 that transmits signals in a wireless manner. Data processing device 500 includes a controller 510. Controller 510 includes a CPU 511 and a storage 512.

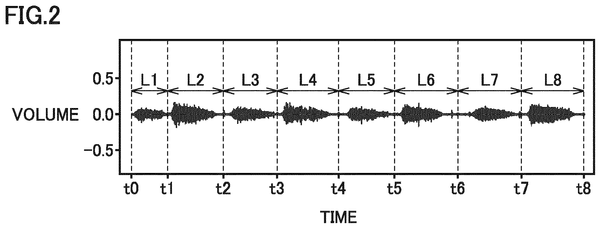

Detector 132 detects respiration signals in a state where detector 132 is attached to a human body and acquires the respiration signals. Detector 132 is, for example, a microphone. In the example in Fig. 1, measurement device 130 including detector 132 is attached to the neck of a subject A. In the present embodiment, the respiration signals are signals indicating volumes of respiratory sound of subject A (see Fig. 2 which will be described later).

The attachment position of measurement device 130 (detector 132) is not limited to the neck, and measurement device 130 may be attached at any position (for example, on the throat or near the ears) of the human body if respiratory sound can be detected. Measurement device 130 may be pasted to the human body or, for example, may be provided in a band that is to be wound around the neck.

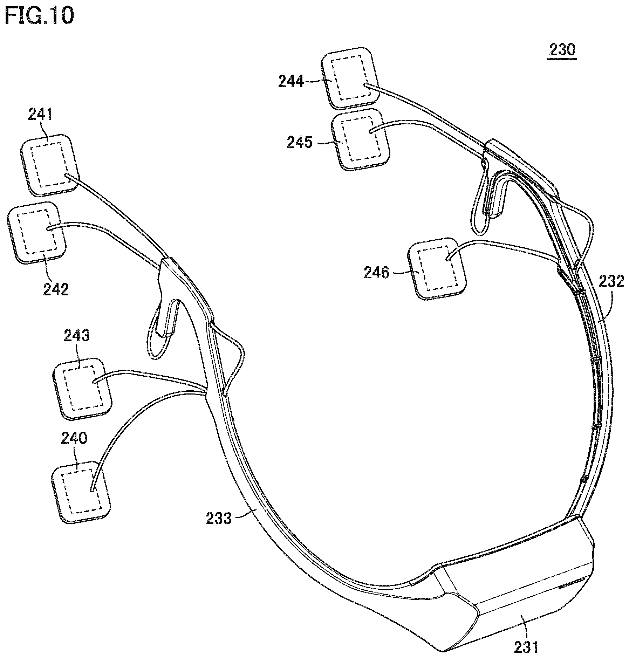

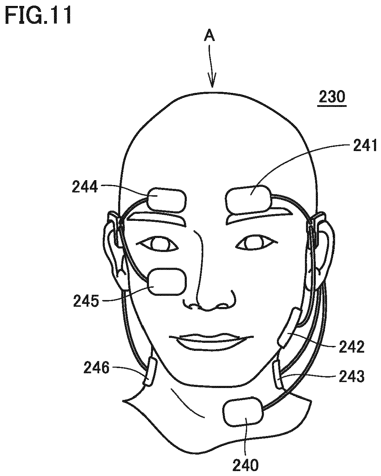

Alternatively, as in a second embodiment (Fig. 10, Fig. 11) which will be described later, the measurement device may be constituted so that a body portion is separate from a sensor. By this means, the measurement device can acquire respiration signals during operation in daily life. For example, it is possible to continuously observe change of respiration during detailed work using the fingers or some kind of work.

CPU 131 controls measurement device 130 as a whole. Storage 133 stores the respiration signals, and the like, acquired by detector 132. CPU 131 causes the respiration signals acquired by detector 132 to be stored in storage 133.

CPU 131 may process the respiration signals before or after the acquired respiration signals are stored to remove vascular sound, ambient sound, and the like, that can be included in the respiration signals. Specific examples of the preprocessing can include a bandpass filter that removes sound in frequency bands other than a frequency band of the respiratory sound.

Further, a memory card 140 that is a removable external storage medium can be inserted into measurement device 130. The respiration signals stored in storage 133 can be taken out by memory card 140 and read by data processing device 500.

Further, measurement device 130 can communicate with data processing device 500. Measurement device 130 can transmit the respiration signals acquired by detector 132 to controller 510 of data processing device 500 in real time. Transmitter 134 transmits the respiration signals stored in storage 133 to controller 510 in a wireless manner.

As described above, data processing device 500 can acquire the respiration signals acquired by detector 132 by reading the respiration signals from memory card 140 or through communication with measurement device 130. In the former method, the acquired respiration signals can be captured by data processing device 500 ex post. In the latter method, the acquired respiration signals can be captured by data processing device 500 in real time.

Controller 510 provided in data processing device 500 controls data processing device 500 as a whole. CPU 131 performs calculation processing and causes a result of the calculation processing to be stored in storage 133.

Controller 510 acquires the respiration signals acquired by detector 132 using the methods as described above. Controller 510 calculates information corresponding to an amount of ventilation by respiration (hereinafter, also referred to as "ventilation amount information") on the basis of the acquired respiration signals.

Then, controller 510 outputs the ventilation amount information. For example, controller 510 may cause the output ventilation amount information to be stored in storage 512, output the ventilation amount information to display device 600 or output the ventilation amount information as communication data to other devices (such as a mobile terminal).

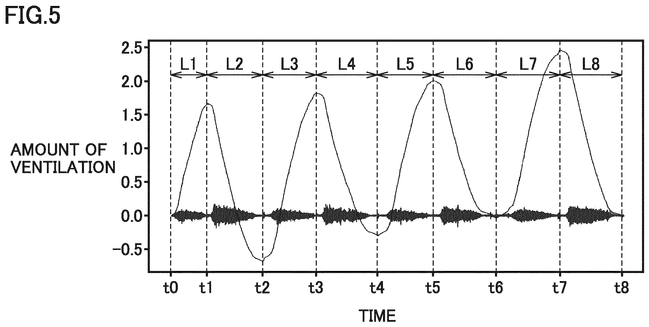

Display device 600 displays the output ventilation amount information. For example, as in Fig. 5 which will be described later, the ventilation amount information is displayed as waveform information. Alternatively, for example, controller 510 may transmit the ventilation amount information as communication data to a mobile terminal (not illustrated) that can communicate with data processing device 500 and display the ventilation amount information on the mobile terminal. Description will be provided in detail below using the drawings in Fig. 2 and subsequent drawings.

Fig. 2 is a graph indicating an example of temporal change of the respiration signals. The respiration signals (volumes of the respiratory sound) acquired by detector 132 are plotted as in, for example, Fig. 2.

Here, in the present embodiment, a signal corresponding to an inspiratory phase will be referred to as an "inspiratory signal" or a "first signal". A signal corresponding to an expiratory phase will be referred to as an "expiratory signal" or a "second signal". In other words, respiration signals acquired when subject A inhales are the inspiratory signals (first signals), and respiration signals acquired when subject A exhales are expiratory signals (second signals).

In the example in Fig. 2, during a period L1 from t0 to 11, subject A inhales, and signals of sound generated by inspiration are acquired. In other words, during period L1, the inspiratory signals (first signals) corresponding to the inspiratory phase are acquired. During a period L2 from t1 to t2, subject A exhales, and signals of sound generated by expiration are acquired. In other words, during period L2, the expiratory signals (second signals) corresponding to the expiratory phase are acquired.

In a similar manner, inspiratory signals are acquired during a period L3 (from t2 to t3), expiratory signals are acquired during period L4 (from t3 to t4), inspiratory signals are acquired during a period L5 (from t4 to t5), expiratory signals are acquired during a period L6 (from t5 to t6), inspiratory signals are acquired during a period L7 (from t6 to t7), and expiratory signals are acquired during a period L8 (from t7 to t8).

Here, it is known that a volume of respiratory sound is proportional to a square of flow velocity of respiration. From this, it is considered that change of a relative amount of ventilation of each phase can be estimated by adding up volumes (respiration signals) of the inspiratory phase and the expiratory phase for each phase and seeing change of the integrated values.

It is assumed in the present embodiment that the ventilation amount information in the inspiratory phase is calculated on the basis of an integrated value of absolute values of the inspiratory signals (first signals), and the ventilation amount information in the expiratory phase is calculated on the basis of an integrated value of absolute values of the expiratory signals (second signals).

Further, it can be determined whether each of the acquired respiration signals is an inspiratory signal (first signal) corresponding to an inspiratory phase or an expiratory signal (second signal) corresponding to an expiratory phase using a publicly known technique. For example, the above can be determined from a difference between a waveform shape of the inspiratory phase and a waveform shape of the expiratory phase.

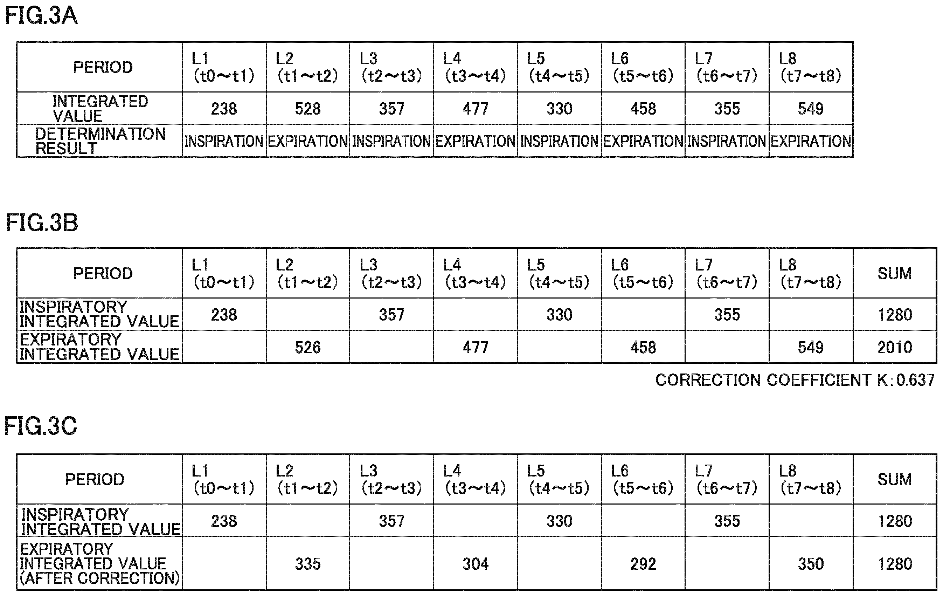

Alternatively, the above can be determined from a difference in frequency characteristics between the inspiratory phase and the expiratory phase. As an example, the following literature A describes that a cumulative power spectrum is greater in the expiratory phase than the inspiratory phase by 500 to 800 dB (Fig. 3A to Fig. 6). In this manner, the inspiratory signal (first signal) and the expiratory signal (second signal) can be extracted from the acquired respiration signals.

[Literature A]: Komorida, and four others, "The correlations of the breath sound with the depth of respiratory and the medical condition of atelectasis", Search report of Nagasaki University School of Engineering, 41(76), pp. 40-45; 2011 <URL: https://web.archive.org/web/20181102022440/http://naosite.lb.nagasakiu.ac.jp/dspace/bitstream/10069/24634/l/41_76_7.pdf>

Fig. 3A to Fig. 3C are views for explaining integrated values of absolute values of the respiration signals and a method for correcting the integrated values. Fig. 3A indicates integrated values of absolute values of inspiratory signals (hereinafter, also referred to as "inspiratory integrated values") or integrated values of absolute values of expiratory signals (hereinafter, also referred to as "expiratory integrated values") calculated during each period of L1 to L8 indicated in Fig. 2. Further, results of determining whether each of L1 to L8 is inspiration or expiration using the methods as described above are indicated.

The inspiratory integrated value indicates an amount corresponding to an amount of ventilation in the inspiratory phase (amount of inhalation). The expiratory integrated value indicates an amount corresponding to an amount of ventilation in the expiratory phase (amount of exhalation).

Here, the amount of inhalation (amount of ventilation in the inspiratory phase) and the amount of exhalation (amount of ventilation in the expiratory phase) are affected by a respiratory condition of the subject in one time of respiration, and thus, are not equal to each other (vary in each time). However, as the number of times of respiration increases, the amount of ventilation in the inspiratory phase should be substantially equal to the amount of ventilation in the expiratory phase. Using such assumption, in the present embodiment, correction as will be described below is performed.

Fig. 3B indicates results obtained by dividing the integrated values in Fig. 3A into the inspiratory integrated values and the expiratory integrated values and obtaining sums of the respective integrated values. The sum of the inspiratory integrated values is 1280, and the sum of the expiratory integrated values is 2010.

According to the above-described assumption, while as the number of times of respiration increases, the sum of the amounts of inhalation should be substantially equal to the sum of the amounts of exhalation, in the above-described example, the expiratory integrated value (2010) is estimated significantly greater than the inspiratory integrated value (1280).

This is because even in a case where the amount of ventilation is the same, a volume measured in the inspiratory phase is not necessarily equal to a volume measured in the expiratory phase due to various reasons such as a difference in way of use of the trachea and the lung. The volumes of the both differ in accordance with a site of subject A to which measurement device 130 is attached. In the present embodiment, measurement device 130 is attached to the neck of subject A, in which case, the volume measured in the expiratory phase becomes greater than the volume measured in the inspiratory phase. In the present embodiment, correction is performed so that the inspiratory integrated value and the expiratory integrated value are balanced using a correction coefficient K.

Specifically, correction coefficient K is set such that correction coefficient K = inspiratory integrated value / expiratory integrated value. In the present example, correction coefficient K = 1280 / 2010 = 0.637. Then, correction is performed by multiplying the expiratory signals by correction coefficient K. In a case where such correction is performed, the expiratory integrated value becomes smaller as in Fig. 3C, and as a result, the sums of both the inspiratory integrated value and the expiratory integrated value become 1280.

Note that in the present embodiment, the volume measured in the expiratory phase is greater than the volume measured in the inspiratory phase, and thus, correction coefficient K < 1. However, it can be assumed that the volume measured in the inspiratory phase is greater than the volume measured in the expiratory phase according to a site of subject A to which measurement device 130 is attached. In this case, correction coefficient K > 1. In a case where the volumes measured in the both phases are balanced, correction coefficient K = 1.

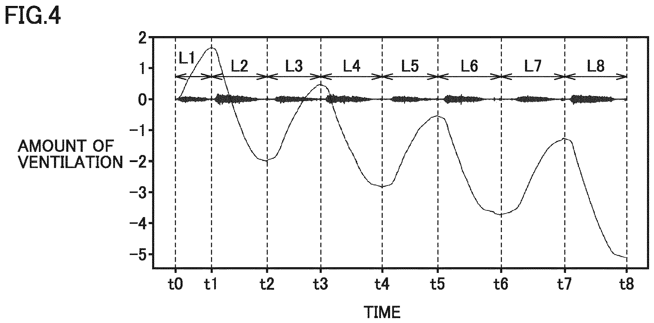

A difference in a calculation result of information corresponding to the amount of ventilation (ventilation amount information) between in a case where correction coefficient K is not used and in a case where correction coefficient K is used will be described below using Fig. 4 and Fig. 5.

In the present embodiment, the ventilation amount information is obtained by adding up absolute values of the inspiratory signals in a case where the respiration signals are inspiratory signals and is obtained by adding up values obtained by multiplying absolute values of the respiration signals by -1 in a case where the respiration signals are expiratory signals. In the following example, description will be provided assuming that the acquired respiration signals are all in positive values.

For example, it is assumed that the respiration signals of (A1, A2, A3, A4) are time-series data, A1 and A2 are inspiratory signals, and A3 and A4 are expiratory signals. In this case, the time-series data of the ventilation amount information before correction becomes (A1, A1 + A2, A1 + A2 - A3, A1 + A2 - A3 - A4).

In a case where correction is performed with correction coefficient K, A3 that is the expiratory signal is corrected to be A3 × K, and A4 is corrected to be A4 × K. As a result, the time-series data of the ventilation amount information corrected with correction coefficient K becomes (A1, A1 + A2, A1 + A2 - A3 × K, A1 + A2 - A3 × K - A4 × K).

Fig. 4 is a graph indicating an example of temporal change of the ventilation amount information before correction. As indicated, during a period of L1 that is a period of the inspiratory signals, the signals are added up as positive values, and thus, the amount of ventilation increases. On the other hand, during a period of L2 that is a period of the expiratory signals, the signals are added up as negative values, and thus, the amount of ventilation decreases.

In a similar manner, the amount of ventilation increases during a period of L3 (inspiratory signals), the amount of ventilation decreases during a period of L4 (expiratory signals), the amount of ventilation increases during a period of L5 (inspiratory signals), the amount of ventilation decreases during a period of L6 (expiratory signals), the amount of ventilation increases during a period of L7 (inspiratory signals), and the amount of ventilation decreases during a period of L8 (expiratory signals).

In this example, there is a relationship of the inspiratory integrated value < the expiratory integrated value. Thus, as in the drawings, the graph is negatively sloped and cannot accurately represent change of the amount of ventilation.

Fig. 5 is a graph indicating an example of temporal change of the ventilation amount information after correction. In a similar manner to Fig. 4, during periods of L1, L3, L4 and L5 that are periods for inspiratory signals, the signals are added up as positive values, and thus, the amount of ventilation increases. On the other hand, during periods of L2, L4, L6 and L7 that are periods for expiratory signals, the signals are added up as negative values, and thus, the amount of ventilation decreases. In this manner, the information corresponding to the amount of ventilation is waveform information.

In this example, there is a relationship of the inspiratory integrated value = the expiratory integrated value after correction. Thus, as in the drawing, the ventilation amount becomes 0 at t8 after L8 ends.

Data processing to be executed by controller 510 will be described next using a flowchart. The data processing is a series of processing to be executed by controller 510 until the respiration signals are acquired and the ventilation amount information is finally output.

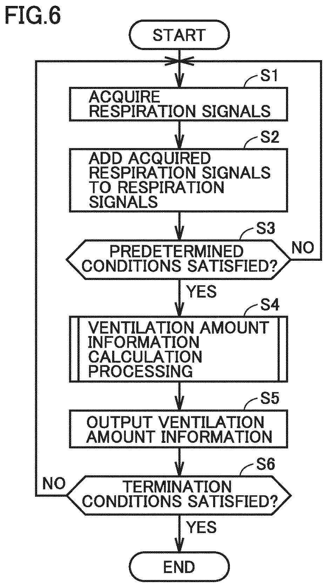

Fig. 6 is a flowchart for explaining the data processing. For example, the data processing is started at a timing at which measurement device 130 and data processing device 500 establish communication and detector 132 starts acquisition of the respiration signals.

When the data processing is started, controller 510 acquires the respiration signals acquired by detector 132 on data processing device 500 side in S1, and the processing proceeds to S2. The respiration signals that have already been acquired are held as respiration signals of time-series data. Controller 510 adds the newly acquired respiration signals to the respiration signals (time-series data) in S2, and the processing proceeds to S3.

Controller 510 determines whether or not predetermined conditions are satisfied in S3. Here, the predetermined conditions are conditions for determining whether the newly acquired respiration signals belong to the inspiratory signals or the expiratory signals.

As described above, whether the respiration signals are signals in the expiratory phase or signals in the inspiratory phase can be determined from waveforms of the respiration signals. Thus, the above determination is preferably made after the predetermined conditions are made to be satisfied at a timing at which the expiratory phase or the inspiratory phase is assumed to end. For example, the predetermined conditions may be made to be satisfied at a timing at which a value of the respiration signals becomes 0 (respiratory sound is interrupted). Alternatively, the predetermined conditions may be made to be satisfied for each period determined in advance.

In a case where it is determined that the predetermined conditions are satisfied (S3: Yes), the processing of controller 510 proceeds to S4. On the other hand, in a case where it is not determined that the predetermined conditions are satisfied (S3: No), controller 510 returns the processing to S1 and continues to acquire the respiration signals until the predetermined conditions are satisfied.

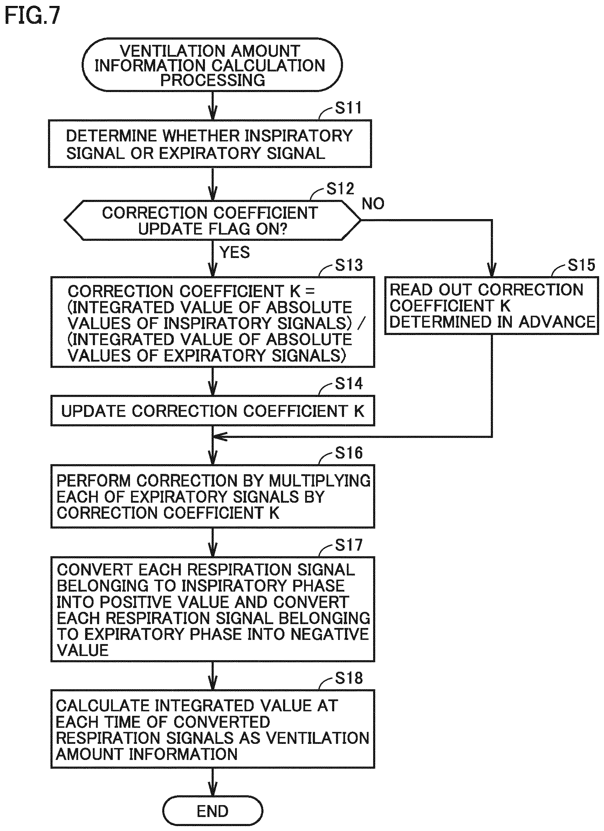

Controller 510 executes ventilation amount information calculation processing in S4, and the processing proceeds to S5. The ventilation amount information is calculated through the ventilation amount information calculation processing. Details thereof will be described later using Fig. 7.

Controller 510 performs processing of outputting the calculated ventilation amount information to display device 600 in S5, and the processing proceeds to S6. Display device 600 displays the ventilation amount information. For example, the ventilation amount information as indicated in Fig. 5 described above is displayed at display device 600.

As described above, even in a case where the respiration signals are acquired, the ventilation amount information cannot be calculated until the predetermined conditions are satisfied. Thus, the ventilation amount information is displayed at display device 600 after a slight time lag instead of being purely displayed in real time.

Controller 510 determines whether or not termination conditions are satisfied in S6. For example, the termination conditions are satisfied in a case where subject A terminates acquisition of the respiration signals at detector 132 or communication with measurement device 130 ends.

In a case where it is determined that the termination conditions are satisfied (S6: Yes), controller 510 ends the data processing. On the other hand, in a case where it is not determined that the termination conditions are satisfied (S6: No), controller 510 returns the processing to S1. In this case, controller 510 further continues to acquire the respiration signals, and calculation processing of the ventilation amount information is performed at a timing at which the predetermined conditions are satisfied next.

Fig. 7 is a flowchart for explaining the ventilation amount information calculation processing. Controller 510 executes the ventilation amount information calculation processing.

When the ventilation amount information calculation processing is started, controller 510 determines whether each of the acquired respiration signals is the inspiratory signal corresponding to the inspiratory phase or the expiratory signal corresponding to the expiratory phase in S11, and the processing proceeds to S12. As described above, whether the signal is a signal corresponding to the expiratory phase or a signal corresponding to the inspiratory signal is determined on the basis of the waveform shape.

Controller 510 determines whether or not a correction coefficient update flag is ON in S12. In the present embodiment, in a case where a value determined in advance is used as the correction coefficient, the correction coefficient update flag is set OFF. On the other hand, in a case where the correction coefficient is determined on the basis of actual measurement values of the respiration signals, the correction coefficient update flag is set ON.

In a case where the correction coefficient update flag is set ON, controller 510 can calculate the ventilation amount information with higher accuracy on the basis of the actual measurement values. On the other hand, in a case where the correction coefficient update flag is set OFF, controller 510 does not calculate the correction coefficient, so that processing load of data processing device 500 can be reduced. Setting of the correction coefficient update flag may be able to be changed by a user, or the correction coefficient update flag may be set ON or OFF in advance for each product.

In a case where it is determined that the correction coefficient update flag is set ON (S12: Yes), the processing of controller 510 proceeds to S13. On the other hand, in a case where it is not determined that the correction coefficient update flag is set ON (S12: No), the processing of controller 510 proceeds to S15.

Controller 510 calculates correction coefficient K = (integrated value of absolute values of inspiratory signals) / (integrated value of absolute values of expiratory signals) in S13, and the processing proceeds to S15. Correction coefficient K is a value obtained by dividing the integrated value of the absolute values of the inspiratory signals among the respiration signals by the integrated value of the absolute values of the expiratory signals among the respiration signals.

The correction coefficient may be calculated using all the measured respiration signals or may be calculated using part of the respiration signals. For example, the correction coefficient may be calculated using all the measured sets, one set including the inspiratory phase + the expiratory phase or may be calculated using a plurality of the latest sets.

Controller 510 updates correction coefficient K in S14, and the processing proceeds to S16. Specifically, controller 510 updates correction coefficient K by replacing correction coefficient K calculated previous time with correction coefficient K calculated this time. In this manner, controller 510 updates correction coefficient K every time the predetermined conditions are satisfied (S3).

Controller 510 reads out correction coefficient K determined in advance in S15, and the processing proceeds to S16. In this case, a value determined in advance is used as correction coefficient K.

Controller 510 performs correction by multiplying each of the expiratory signals among the respiration signals by correction coefficient K in S16, and the processing proceeds to S17. On the other hand, controller 510 does not perform correction on the inspiratory signals among the respiration signals. In this manner, controller 510 performs calculation different between the inspiratory signals and the expiratory signals among the respiration signals.

Controller 510 converts each respiration signal belonging to the inspiratory phase into a positive value and converts each respiration signal belonging to the expiratory phase into a negative value in S17, and the processing proceeds to S18. Specifically, controller 510 converts the respiration signals belonging to the inspiratory phase into positive values by obtaining absolute values of the respiration signals (inspiratory signals). Controller 510 converts the respiration signals belonging to the expiratory phase into negative values by multiplying absolute values of the respiration signals (expiratory signals) by -1. Controller 510 calculates the integrated values of the converted respiration signals at each time as the ventilation amount information in S18 and ends the ventilation amount information calculation processing.

In this manner, controller 510 calculates information corresponding to the amount of ventilation by respiration on the basis of the extracted inspiratory signals and expiratory signals. Specifically, controller 510 calculates the information corresponding to the amount of ventilation by performing correction using correction coefficient K and adding up the respiration signals while one of the inspiratory signals and the expiratory signals is in positive values and the other is in negative values.

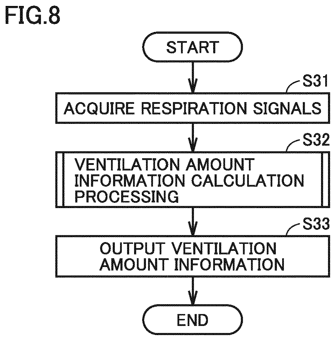

Fig. 8 is a flowchart for explaining data processing according to a modification. The data processing in the present modification assumes a scene in which the data processing is executed ex post after measurement of all the respiration signals is completed.

For example, after all measurement is completed, the user causes the respiration signals to be stored in memory card 140. Then, the user causes data processing device 500 to read the memory card 140. The data processing is started using the respiration signals on memory card 140 by operation by the user.

When the data processing is started, controller 510 acquires all the respiration signals on memory card 140 in S31, and the processing proceeds to S32.

Controller 510 executes the ventilation amount information calculation processing (see Fig. 7) in S32, and the processing proceeds to S33. In this case, controller 510 only requires to calculate correction coefficient K while setting the correction coefficient update flag = ON and targeting at all the respiration signals.

Controller 510 performs processing of outputting the calculated ventilation amount information to display device 600 in S33 and ends the data processing. Display device 600 displays the ventilation amount information. For example, the ventilation amount information as indicated in Fig. 5 described above is displayed at display device 600.

As described above, in the present embodiment, the information corresponding to the amount of ventilation is calculated on the basis of the respiration signals acquired by detector 132 attached to the human body. A device that estimates an amount of ventilation by respiration by a detector being attached to a human body includes a belt type respirometer disclosed in the following literature B.

[Literature B] Zero C Seven, Inc., pulmonary respiration transducer: TSD201, http://biopac-sys.jp/products/tsd201/
The belt type respirometer detects change in a length of a belt wound around the breast by respiration and estimates an amount of ventilation from stereoscopic change of the breast. However, the belt type respirometer provides strong feeling of constraint of the breast, and thus, it is hard to say that the amount of ventilation is measured in an environment where the subject can freely move.

In the present embodiment, by controller 510 outputting the information corresponding to the amount of ventilation calculated on the basis of the respiration signals acquired by detector 132 attached to the human body, it is possible to easily measure data also in an environment where the subject can freely move. Further, by controller 510 performing calculation on each of the respiration signals, the calculation being different between when the signal is the inspiratory signal and when the signal is the expiratory signal, it is possible to calculate the information corresponding to the amount of ventilation while taking into account a difference in characteristics between the expiratory signal and the inspiratory signal. This makes it possible to easily grasp the information corresponding to the amount of ventilation also in an environment where the subject can freely move.

### [Second embodiment]

In the first embodiment, a configuration including one detector is employed. However, the present disclosure is not limited to this, and a configuration including a plurality of detectors may be employed. Hereinafter, points different from the first embodiment will be described, and description of common parts will be omitted.

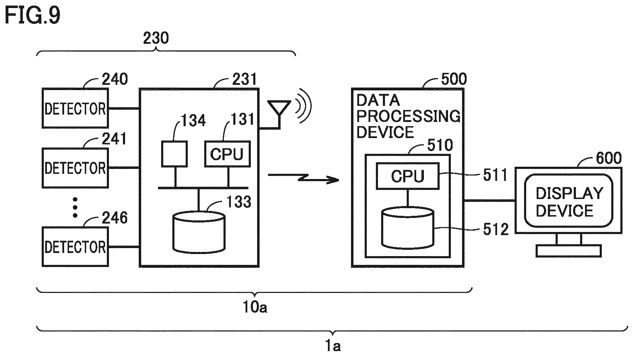

Fig. 9 is a view illustrating an example of an overall configuration of a respiratory measurement system 1a according to a second embodiment. Respiratory measurement system 1a includes a respiratory measurement device 10a and display device 600. Respiratory measurement device 10a includes data processing device 500 and a measurement device 230. Data processing device 500 and display device 600 are the same as those in the first embodiment.

In the second embodiment, detectors 240 to 246 are provided as detectors other than CPU 131, storage 133 and transmitter 134. Fig. 10 is a view illustrating an example of measurement device 230. Measurement device 230 includes a body portion 231, arms 232 and 233, and detectors 240 to 246.

Arm 232 and arm 233 respectively extend from both ends of body portion 231. Each of arm 232 and arm 233 has a shape curving and extending so as to be along the head of subject A in a state where measurement device 230 is worn.

The arm 232 and the arm 233 extend so as to form substantially semicircles. Portions of arcs of the substantially semicircles are hung on the ears of subject A. Body portion 231 is connected to each of detectors 240 to 246 with wirings.

Fig. 11 is a view for explaining a state where measurement device 230 is worn on the human body (subject A). Detector 240 acquires the respiration signals in a similar manner to detector 132 in the first embodiment. Further, detectors 241 to 246 acquire biological signals such as myopotentials of the face, a body temperature and a heart rate.

In this example, detectors 241, 242, 244 and 245 are attached to the face of subject A. These sensors are attached to, for example, zygomatic muscles or corrugator muscles and acquire myopotentials of the zygomatic muscles or myopotentials of the corrugator muscles. Detector 243 is a sensor that measures a heart rate of subject A. Detector 246 is a sensor that acquires a body temperature of subject A.

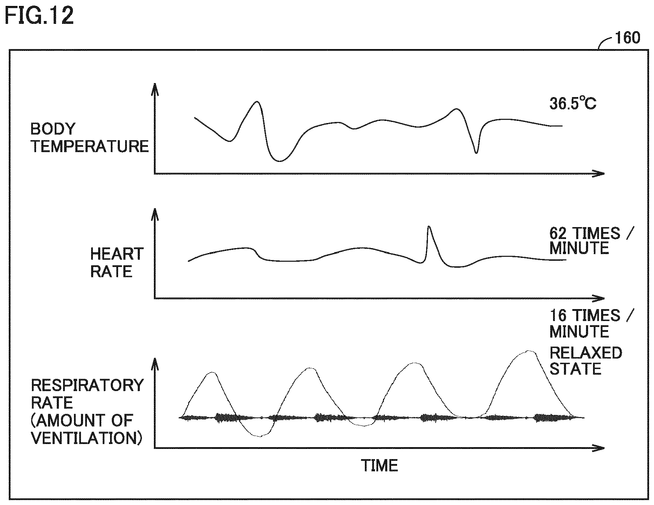

Fig. 12 is a view illustrating a display example of output information. Controller 510 can output biological signals different from the ventilation amount information along with the ventilation amount information. For example, the biological signals such as the body temperature and the heart rate described above are output as the biological signals.

Temporal change of the body temperature is indicated as a graph on a screen 160 of display device 600. Along with this, a current body temperature of 36.5 °C is displayed on screen 160. Further, temporal change of the heart rate is indicated as a graph on screen 160. Along with this, a current heart rate of 62 / minute is displayed on screen 160.

Further, the ventilation amount information is displayed on screen 160. A graph of a signal waveform is displayed as the ventilation amount information in the drawing. Along with this, a current respiratory rate of 16 times / minute is displayed on screen 160.

The respiratory condition of the subject can be grasped from the ventilation amount information. For example, by observing the signal waveform, it is possible to grasp a deep and slow respiratory condition or a shallow and rapid respiratory condition. In a case of the deep and slow respiratory condition, it can be determined that the subject is in a relaxed state. Other than this, a nervous state, a concentrated state, or the like, can be determined as the biological information.

In this manner, the biological information such as the "relaxed state", the "nervous state" and the "concentrated state" can be calculated and obtained from the signal waveform. The biological information indicated in the present embodiment is information indicating a state of autonomic nerves. On screen 160, information indicating the "relaxed state" is displayed as the biological information. In this manner, from the acquisition results of detector 240 that acquires the respiration signals, one signal waveform may be generated or information indicating the state of autonomic nerves may be generated.

Further, data processing device 500 can estimate a feeling corresponding to the four myopotential muscles using a table in which the above-described four myopotential muscles are associated with feelings. Information estimated in this manner may be displayed on screen 160.

### [Aspect]

(First item) A respiratory measurement method according to one aspect includes acquiring respiration signals by a detector attached to a human body, extracting, from the acquired respiration signals, a first signal and a second signal indicating an expiratory phase, and calculating information corresponding to an amount of ventilation by respiration on the basis of the extracted first signal and second signal. The calculating includes performing, for each of the respiration signals, different operation depending upon whether the signal is the first signal or the second signal.

According to the respiratory measurement method according to the first item, different operation is performed, for each of the respiration signals, depending upon whether the signal is a first signal indicating an inspiratory phase or a second signal indicating an expiratory phase. By this means, it is possible to easily grasp the information corresponding to the amount of ventilation also in an environment where the subject can freely move.

(Second item) In the respiratory measurement method according to the first item, the information corresponding to the amount of ventilation includes waveform information.

According to the respiratory measurement method according to the second item, by confirming the waveform information as the information corresponding to the amount of ventilation, it is possible to easily grasp the information corresponding to the amount of ventilation also in an environment where the subject can freely move.

(Third item) In the respiratory measurement method according to the first item, the information corresponding to the amount of ventilation includes biological information obtained through calculation.

According to the respiratory measurement method according to the third item, by confirming the biological information as the information corresponding to the amount of ventilation, it is possible to easily grasp the information corresponding to the amount of ventilation also in an environment where the subject can freely move.

(Fourth item) In the respiratory measurement method according to any one of the first to the third items, the calculating includes performing correction by multiplying the second signal among the respiration signals by a correction coefficient, and calculating the information corresponding to the amount of ventilation by integrating the respiration signals while one of the first signal and the second signal is in a positive value and the other is in a negative value.

According to the respiratory measurement method according to the fourth item, by performing correction by multiplying each of the second signal by the correction coefficient, it is possible to correct an error due to a difference in characteristics between the first signal corresponding to the inspiratory phase and the second signal corresponding to the expiratory phase.

(Fifth item) In the respiratory measurement method according to the fourth item, the correction coefficient is a value obtained by dividing an integrated value of an absolute value of the first signal among the respiration signals by an integrated value of an absolute value of the second signal among the respiration signals.

According to the respiratory measurement method according to the fifth item, it is possible to correct an error while making a sum of an amount of ventilation by inhalation the same as a sum of an amount of ventilation by exhalation.

(Sixth item) The respiratory measurement method according to the fifth item further includes updating the correction coefficient every time predetermined conditions are satisfied.

According to the respiratory measurement method according to the sixth item, by updating the correction coefficient while reflecting the newly acquired respiration signals, it is possible to calculate the information corresponding to the amount of ventilation with higher accuracy.

(Seventh item) In the respiratory measurement method according to the fourth item, the correction coefficient is set to be a predetermined value.

According to the respiratory measurement method according to the seventh item, it is possible to reduce calculation load for obtaining the correction coefficient.

(Eighth item) The respiration measurement method according to any one of the first to the seventh items further includes outputting the calculated information corresponding to the amount of ventilation, and the outputting includes outputting a biological signal different from the information corresponding to the amount of ventilation along with the information corresponding to the amount of ventilation.

According to the respiratory measurement method according to the eighth item, it is possible to grasp biological information also from information different from the information corresponding to the amount of ventilation as well as the information corresponding to the amount of ventilation.

(Ninth item) A respiratory measurement device according to another aspect includes a detector that acquires respiration signals in a state where the detector is attached to a human body, and a controller that processes the respiration signals. The controller extracts, from the acquired respiration signals by the detector, a first signal indicating an inspiratory phase and a second signal indicating an expiratory phase. The controller calculates information corresponding to an amount of ventilation by respiration on the basis of the extracted first signal and second signal. The controller performs, for each of the respiration signals, different operation depending upon whether the signal is the first signal or the second signal.

According to the respiratory measurement device according to the ninth item, different operation is performed, for each of the respiration signals, depending upon whether the signal is a first signal indicating an inspiratory phase or a second signal indicating an expiratory phase. By this means, it is possible to easily grasp the information corresponding to the amount of ventilation also in an environment where the subject can freely move.

(Tenth item) In the respiratory measurement device according to the ninth item, the detector includes a storage that stores the acquired respiration signals and a transmitter that transmits the respiration signals stored in the storage to the controller in a wireless manner.

According to the respiratory measurement device according to the tenth item, by employing a configuration where the detector that acquires the respiration signals performs communication with the controller that processes the respiration signals in a wireless manner, it is possible to easily grasp the information corresponding to the amount of ventilation also in an environment where the subject can freely move.

(Eleventh item) A respiratory measurement system according to another aspect includes the respiratory measurement device according to the ninth or the tenth item, and a display device. The controller outputs the calculated information corresponding to the amount of ventilation. The display device displays the output information corresponding to the amount of ventilation.

According to the respiratory measurement system according to the eleventh item, calculation is performed on each signal of the respiration signals, the calculation being different between when the signal is the first signal corresponding to the inspiratory phase and when the signal is the second signal corresponding to the expiratory phase. By this means, it is possible to easily grasp the information corresponding to the amount of ventilation also in an environment where the subject can freely move.

It should be understood that the embodiments disclosed herein are by way of illustration and example only in all aspects and are not to be taken by way of limitation. The scope of the present invention is not indicated by the above description but indicated by the claims and is intended to include all changes within the content and the range equivalent to the claims.

### REFERENCE SIGNS LIST

1, 1a Respiratory measurement system; 10, 10a Respiratory measurement device; 130, 230 Measurement device; 131 CPU; 132, 240 to 246 Detector; 133 Storage; 134 Transmitter; 140 Memory card; 160 Display screen; 231 Body portion; 232, 233 Arm; 500 Data processing device; 510 Controller; 511 CPU; 512 Storage; 600 Display device

## Claims

1. A respiratory measurement method comprising:
acquiring respiration signals by a detector attached to a human body;
extracting, from the acquired respiration signals, a first signal indicating an inspiratory phase and a second signal indicating an expiratory phase; and
calculating information corresponding to an amount of ventilation by respiration on a basis of the extracted first signal and second signal,
wherein the calculating includes performing, for each of the respiration signals, different operation depending upon whether the signal is the first signal or the second signal.

2. The respiratory measurement method according to claim 1,
wherein the information corresponding to the amount of ventilation includes waveform information.

3. The respiratory measurement method according to claim 1,
wherein the information corresponding to the amount of ventilation includes biological information obtained through calculation.

4. The respiratory measurement method according to claim 1,
wherein the calculating includes:
performing correction by multiplying the second signal among the respiration signals by a correction coefficient; and
calculating the information corresponding to the amount of ventilation by integrating the respiration signals while one of the first signal and the second signal is in a positive value and the other is in a negative value.

5. The respiratory measurement method according to claim 4,
wherein the correction coefficient is a value obtained by dividing an integrated value of an absolute value of the first signal among the respiration signals by an integrated value of an absolute value of the second signal among the respiration signals.

6. The respiratory measurement method according to claim 5, further comprising:
updating the correction coefficient every time predetermined conditions are satisfied.

7. The respiratory measurement method according to claim 4,
wherein the correction coefficient is set to be a predetermined value.

8. The respiratory measurement method according to claim 1, further comprising:
outputting the calculated information corresponding to the amount of ventilation,
wherein the outputting includes outputting a biological signal different from the information corresponding to the amount of ventilation along with the information corresponding to the amount of ventilation.

9. A respiratory measurement device comprising:
a detector that acquires respiration signals in a state where the detector is attached to a human body; and
a controller that processes the respiration signals,
wherein the controller
extracts, from the acquired respiration signals by the detector, a first signal indicating an inspiratory phase and a second signal indicating an expiratory phase,
calculates information corresponding to an amount of ventilation by respiration on a basis of the extracted first signal and second signal, and
performs, for each of the respiration signals, different operation depending upon whether the signal is the first signal or the second signal.

10. The respiratory measurement device according to claim 9,
wherein the detector includes:
a storage that stores the acquired respiration signals; and
a transmitter that transmits the respiration signals stored in the storage to the controller in a wireless manner.

11. A respiratory measurement system comprising:
the respiratory measurement device according to claim 9; and
a display device,
wherein the controller outputs the calculated information corresponding to the amount of ventilation, and
the display device displays the output information corresponding to the amount of ventilation.
